# EUROPEAN PATENT SPECIFICATION

(11) **EP 4 774 609 B1**
(45) Date of publication and mention of the grant of the patent: **07.10.2026**
(21) Application number: 25810969.3
(22) Date of filing: 31.10.2025
(51) Int. Cl.: G06F 3/01, A61B 3/113, A61B 5/16, G02B 27/00, G02B 27/01, G06N 20/00, G06N 3/02, G06F 1/16, G06F 1/3231, G06F 1/3234, G06N 3/00, G06N 3/045, G06N 3/048, G06N 3/063, G06N 3/08, G06N 3/082, G06N 3/084, G06N 5/01, G06N 20/10

(54) **UNIVERSAL AND POWER-EFFICIENT EYE-TRACKING DEVICE FEATURING A COMFORTABLE FIT FOR DIFFERENT HEAD SHAPES AND SIZES**
UNIVERSELLE UND LEISTUNGSEFFIZIENTE AUGENVERFOLGUNGSVORRICHTUNG MIT KOMFORTABLER PASSFORM FÜR UNTERSCHIEDLICHE KOPFFORMEN UND -GRÖSSEN
DISPOSITIF DE SUIVI OCULAIRE UNIVERSEL ET ÉCONOME EN ÉNERGIE OFFRANT UN AJUSTEMENT CONFORTABLE POUR DIFFÉRENTES FORMES ET TAILLES DE TÊTE

(30) Priority: 21.11.2024 WO PCT/EP2024/083187; 28.08.2025 WO PCT/EP2025/074578
(43) Date of publication of application: 15.07.2026
(73) Proprietor: Viewpointsystem GmbH, 1010 Wien (AT)
(72) Inventor: VINDIGNI, Eddy, 1020 Wien (AT); LINSENMAIER, Frank, 1070 Wien (AT); CHROMY, Patrick, 1020 Wien (AT); ORTS, Javier, 1160 Wien (AT); SCHMIED, Roman, 1020 Wien (AT)
(74) Representative: Schardmüller Gall-Schuhmann Patentanwälte OG
(86) International application number: PCT/EP2025/081603
(87) International publication number: WO 2026/109282

(56) References cited:
- WO-A1-2022/271639
- US-A1- 2021 011 288
- US-A1- 2021 116 995
- US-A1- 2024 377 648

## Description

### Technical Field

A head-mounted device embedding an eye descriptor unit refers to a specialized electronic system designed to detect, measure, and analyze ocular movements with a certain degree of accuracy. These devices, typically comprising a combination of eye sensors, for example, imaging cameras, and software algorithms, monitor the position, movement, and gaze direction of the user's eyes. By capturing data on where a user is looking, an eye-tracking device provides insights into attention, interest, and visual engagement, which may be applied across various fields, including assistive technologies, behavioral research, user interface optimization, and immersive virtual reality systems.

Due to their ability to non-invasively and accurately interpret human visual attention, eye-tracking devices are increasingly integrated into technologies for medical diagnostics, gaming, advertising analytics, and human-computer interaction.

So-called head-mounted eye-tracking technology refers to a class of wearable systems specifically designed to track eye movements from a device affixed directly to the user's head (so-called head-mounted device). These devices are generally more accurate in dynamic environments, as they move in concert with the user's head, maintaining a consistent viewpoint for tracking ocular activity. Head-mounted eye/gaze trackers offer a range of designs, each leveraging different configurations of sensors, cameras, and optical components to suit varied application requirements.

Eye-trackers integrated into a wearable device are also known. Generally, such eye-trackers are built into head-mounted displays (HMDs), such as virtual reality (VR) or augmented reality (AR) headsets. These systems employ small cameras or optical sensors embedded within the display near the lenses, allowing for real-time gaze tracking alongside immersive visual experiences. They are integral in VR and AR applications where the user's gaze must be continuously tracked to enhance interactive elements, improve rendering techniques (such as foveated rendering), or study user behavior within a virtual environment.

Smart glasses with embedded eye-tracking technology represent a subset of head-mounted devices where eye-tracking components are integrated into eyewear that resembles traditional glasses. These systems are typically lightweight and designed for extended wear, making them ideal for applications requiring unobtrusive, long-duration tracking, such as behavioral research in naturalistic settings or real-world advertising studies. Smart glasses utilize miniature cameras or sensors, usually embedded within the frame, to track eye movement with minimal impact on the wearer's mobility.

Head-mounted eye-tracking devices utilize a range of specialized processors to support the demanding requirements of eye data processing, encompassing real-time gaze detection, tracking, and data interpretation. The processors integrated within these devices-namely central processing units (CPUs), graphics processing units (GPUs) and application-specific integrated circuits (ASICs)-each contribute unique computational advantages tailored to the requirements of continuous, high-precision eye tracking.

Central processing units (CPUs) provide foundational control and general-purpose computing within the device, managing system operations and executing control algorithms that enable seamless interaction between various processing units. The CPU often coordinates the input from optical sensors or cameras and ensures that data is routed efficiently for further processing.

For the computationally intensive tasks of image analysis and real-time data visualization associated with eye tracking, many systems incorporate graphics processing units (GPUs). GPUs are highly suited for parallel processing, a requirement in high-resolution image capture and processing, where rapid computation is essential to identify and interpret eye features accurately. This capability is particularly beneficial in applications such as virtual and augmented reality, where immediate and precise gaze mapping enhances user experience and interactivity.

**In** scenarios that demand exceptionally high processing speed and minimal delay, particularly in specialized or medical applications, application-specific integrated circuits (ASICs) may be incorporated. ASICs are custom designed to perform eye data processing functions, delivering high accuracy through dedicated circuitry tailored to the exact computational demands of eye tracking.

By integrating CPUs for system control, GPUs for image processing and ASICs for specialized performance, head-mounted eye-tracking devices achieve the comprehensive processing capabilities required for accurate, real-time gaze tracking across a variety of environments and applications.

### Prior Art

The European Patent EP 4 281 829 describes an eye-tracking kit designed specifically for integration with regular eyeglasses. This invention targets the field of wearable technology, specifically enhancing user interaction and experience by monitoring eye movement and gaze direction. The design outlines a modular system that includes an eye-tracking component fitted onto or embedded within the frame of eyeglasses. The system consists of multiple elements, such as sensors, processors, and communication units, optimized for minimal size to ensure comfort and usability for extended wear.

One of the key features highlighted in the patent is the emphasis on non-intrusive user experience, whereby the eye-tracking kit can be seamlessly attached to conventional eyewear without requiring extensive modifications. This adaptability aims to make eye-tracking more accessible and practical for various applications, including augmented reality (AR), virtual reality (VR), and user interface control systems.

Furthermore, EP 4 399 562 describes a nose pad eye tracking module designed to attach easily to smart glasses or head-wearable devices that do not have nose pads. This module has two main parts: a U-shaped first part with arms acting as customizable nose pads housing left and right eye sensors to track the user's pupil positions, and a second part with a mechanical interface on top that connects securely from below to the central portion of the smart glasses. The module includes electronic components like data processing, memory, and communication interfaces to send eye movement information to an external computer device. This claim protects the specific design of an integrated, readyto-use eye tracking module that replaces traditional nose pads on smart glasses while providing a stable mechanical and electrical connection from underneath.

However, both EP 4 281 829 and EP 4 399 562 are NOT focused on reducing the eye-tracking unit's power consumption, improving response times, or saving bandwidth for the unit itself. Furthermore, both the disclosures do not address ergonomic factors of the head-mounted eye-tracking devices with respect to different head-sizes and shapes. In addition, EP 4 399 562, even if it mentions that the processing unit in the eye-tracking module may be designed as a combination of a microcontroller or processor together with memory, the specification does not mention any use of a machine learning hardware accelerator and the disclosed module is configured to perform video recording and only the initial analysis of eye images and not any full computation of any array of eye descriptor of the user.

For the sake of clarity, eye-tracking system architectures designed to enhance the integration and performance of electronic systems, such as AR/VR/XR headsets, are known. Generally, these eye-tracking architectures include the use of a general-purpose chipset, characterized by the incorporation of multi-core processing capabilities for processing the eye data directly in the AR/VR headset. Processing power for AR applications is generally managed within the headset itself and requires external computing support for more complex tasks. Such a kind of eye-tracking architecture is heavily power-consuming and subject to a certain degree of latency, and harms real-time eye-data processing.

### Purposes of the Invention

One objective of the present invention, according to a first of its aspects, is obtaining a head-mounted device, implementing an eye descriptor unit, capable of executing eye/eye descriptor computations with minimal energy consumption, that can be connected to many possible computer application devices, therefore not only VR/XR headsets as computer application devices but also smart glasses like AR glasses with minimalistic hardware structure. This approach allows the unit to support real-time eye-tracking functionalities while maintaining a low power profile, optimized computational efficiency, and reduced heating dispersion issues, thus enhancing the overall usability of the eye descriptor unit.

A second objective of the present invention is to provide a head-mounted device embedding an eye descriptor unit able to compute the complete eye descriptor data in the eye descriptor unit itself, reducing the unnecessary traffic to the central repository of the computer application device, which is the recipient of the user's eye descriptor data already processed.

A third objective of the present invention is to provide a head-mounted device, embedding an eye descriptor unit that may not interfere with the user's nose, and/or with the user's field of view, with a sleek and streamlined design, adaptive to fit different head shapes and sizes and comfortable to be worn by different ethnics group, considering their typical anatomical traits.

A fourth objective of the present invention is to provide a head-mounted device, embedding an eye descriptor unit, with optimized manufacturing costs and at the same time a solid structure in the nose part frame where the eye sensors are arranged.

A further objective of the present invention is to provide an eye descriptor unit that is very lightweight, minimizing the total weight of the computer application device.

Another objective of the present invention is to provide a method for detecting user eye descriptors using a head-mounted device embedding an eye descriptor unit, being the method robust and accurate enough to estimate and calculate the user eye descriptors in real time.

Another objective of the present invention is to provide a method for detecting a complete array of user eye descriptors using a head-mounted device embedding an eye descriptor unit according to the aspects mentioned above.

### Summary of the Invention

Hereinafter are summarized some technical aspects of the present inventions, which enable some of the most important purposes to be achieved.

According to a first aspect, this invention relates to a head-mounted device embedding an eye descriptor unit for estimating the user's gaze when worn by a user, said eye descriptor unit comprising at least one eye sensor facing the user's left eye and one eye sensor facing the user's right eye, an edge computation unit provided with a machine learning hardware accelerator support and configured to receive as input unprocessed user's eye representation data from the eye sensors and to send as output an array of user's eye-descriptors when in use and wherein the edge computation unit is programmed with a customized bare metal code or with Real-Time Operating System RTOS for microcontrollers or with a combination of customized bare metal code and Real-Time Operating System RTOS for microcontrollers.

Such a unit is very compact and particularly low power-consuming, optimized to run the entire eye-tracking pipeline or any user eye descriptor pipeline directly in the edge computing unit, receiving as input unprocessed data from the eye sensors and outputting small-sized processed data files or in real-time data streams.

The edge computation unit is programmed with a customized bare metal code or with Real-Time Operating System RTOS for microcontrollers or with a combination of customized bare metal code and Real-Time Operating System RTOS for microcontrollers, entailing no OS (Operative System) Overhead. The absence of background tasks or OS-induced wakeups means, that the eye descriptor unit according to the present invention consumes power only for tasks explicitly defined by the customized bare metal code or Real-Time Operating System RTOS for microcontrollers or both of the edge computing unit processing data directly from the eye sensors.

According to a second aspect, this invention relates to a head-mounted device embedding an eye descriptor unit wherein the edge computed unit is configured to implement a method accurate and robust enough to predict user's eye descriptors.

According to a third aspect, this invention relates to a head-mounted device embedding an eye descriptor unit with a sleek and streamlined designed, without any protruding part in the nose frame area interfering with the user's field of view.

According to a fourth aspect, this invention relates to a head-mounted device embedding an eye descriptor unit designed and structured in such a way that it is comfortable to be worn by most different anatomical ethnics traits of ethnic groups, and able to fit different head sizes and shapes.

According to a further aspect, this invention relates to a method for detecting user's eye descriptors using the head-mounted device embedding the eye descriptor unit, according to what is claimed in the dependent claims of the present specification.

### Brief Description of Drawings

The structural and functional features of the present invention and its advantages with respect to the known prior art will become even clearer from the underlying claims, and in particular from an examination of the following description, made with reference to the attached figures, which show a preferred but not limited schematic embodiment of the invented method, system, device, in which:
Figure 1 illustrates the head-mounted eye-tracking device in a 3D view according to the present invention.
Figure 1A illustrates the eye-tracking descriptor unit according to the present invention.
Figure 2 illustrates the nose bridge frame of the head-mounted eye-tracking device in a 3D view with the reference system used in the present invention.

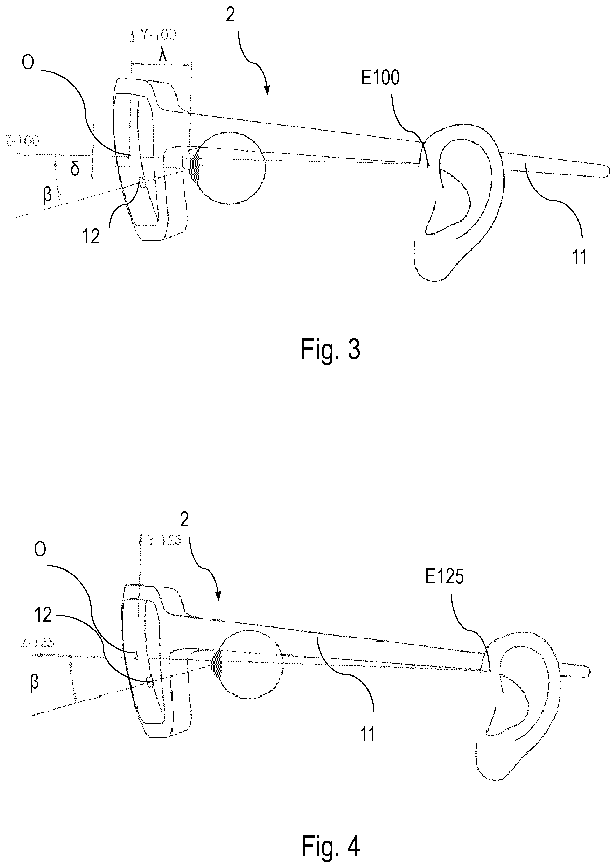

Figures 3 and 4 illustrate the impact of different head sizes on the length of the temples of the head-mounted eye-tracking device according to the present invention.

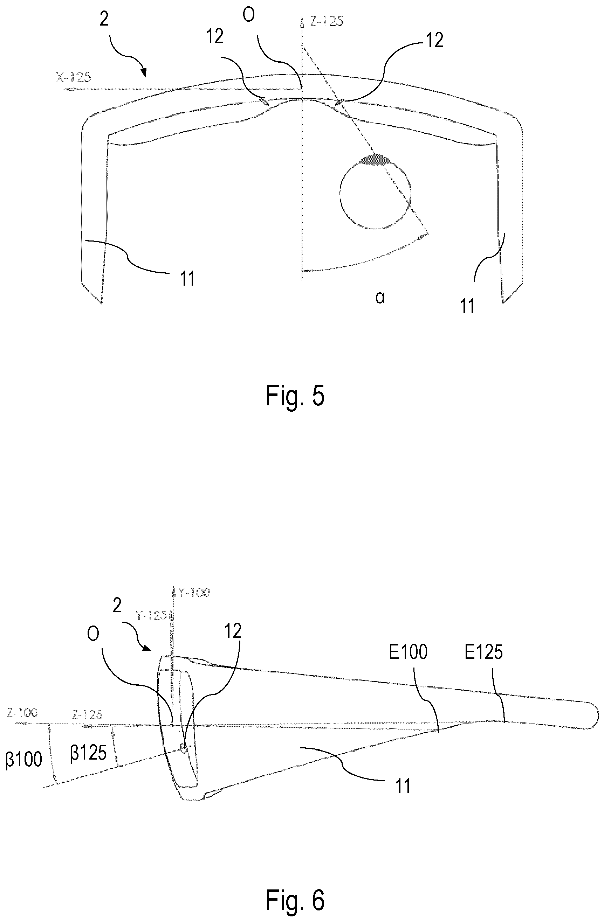

Figure 5 is a top view of the head-mounted eye-tracking device according to the present invention, with indication of angle alpha in the plane XY.

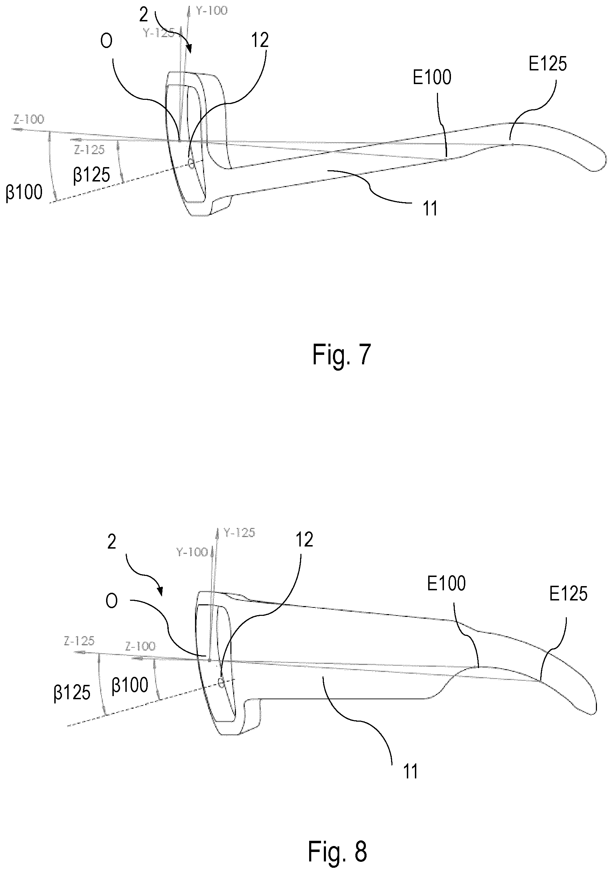

Figures 6 to 8 show how different temple form-factors, due to embedded electronics or the style of the device, influence the eye sensor orientation angle.

### Modes for Carrying out the Invention

The present invention discloses a head-mounted universal and power-efficient eye-tracking device featuring a comfortable fit for different head shapes and sizes, which embeds an edge-computed eye descriptor unit and a method for processing a user's eye descriptors using the head-mounted eye-tracking device.

The specifications "right" or "left" or "high" or "low" or "front" or "back" relate to the intended manner of wearing eyeglasses and the eye tracking module by a human being.

A spectacle frame, as commonly employed in corrective and non-corrective eyewear, generally comprises a pair of eye-rims (also referred to as rims or eye wires), a nose bridge, a pair of endpieces, two temples (or temple arms), and associated hinges and fasteners.

The eye rims are generally constructed as annular or substantially closed structures adapted for securely receiving, retaining, and supporting corrective or protective lenses. Each eye rim features an inner circumferential groove or seat dimensioned for accommodating the edge of a lens. The rims are configured to maintain said lens in a fixed position relative to the frame structure.

Interconnecting the pair of eye-rims is the nose bridge (or bridge), which functions to support the spectacle frame upon the user's nasal structure. The nose bridge may be formed as a unitary member with the eye-rims or attached thereto via integrally molded or mechanically secured joints. The bridge may incorporate, or be associated with, a pair of nose pads or nose pad arms, each adapted to contact the lateral surface of the user's nose for improved comfort, retention, and weight distribution. The bridge may be of a keyhole type, saddle type, or any other conventional or structurally advantageous configuration.

The temples are structurally contiguous with the lateral portions of each eye-rim and serve as mounting regions for the hinges. The hinges provide pivotal engagement between each endpiece and the corresponding temple, permitting the temples to articulate between an open (wearing) configuration and a closed (storage) configuration. The temples are elongated, typically arcuate members dimensioned to extend rearwardly from the frame, over the ears of the wearer, thereby stabilizing and retaining the glasses in the operative position. The temples are designed in such a way that the points that are assumed to be in contact with the top of the ear of the user, are often recognizable because the temple shape bends downwards.

All the spectacle frame parts mentioned above are reflected in head-mounted eye-tracking devices having the structure of spectacles.

Eye-tracking glasses may have a part of their frame to retain the lenses, which may be prescription lenses or sun lenses or specific filtering/protective lenses. They have a portion, so-called nose bridge, acting as a support for the eye-tracking device on the user's nose once in use.

Nowadays are known VR/AR/XR headsets and so-called smart glasses. In some cases, these devices have a very minimal spectacle frame, including an upper part provided with an element constituting the nose frame, in order to have the device placed stably and supported by the nose of the user.

VR headsets are designed to fully immerse users in a computer-generated environment, separating them from the physical world and creating a realistic virtual space. Their main components include high-resolution OLED or LC display panels positioned close to the eyes, providing stereoscopic 3D images by showing slightly different visuals to each eye. To enhance this immersion, VR headsets use lenses that focus and reshape images. The computing power required for VR can be provided by an external computer or, in standalone devices, through onboard processors.

In contrast, AR headsets are designed to overlay digital information onto the real world, providing a mixed-reality experience that enables users to view and interact with virtual objects within their physical environment. These headsets include transparent or semi-transparent display systems, like optical waveguides or reflective displays, that blend digital imagery with the real world by projecting images through micro-projectors onto the lenses. To anchor these virtual images effectively, AR headsets use a combination of lenses and optics to ensure clear and distortion-free visuals. Advanced AR headsets may rely on Simultaneous Localization and Mapping (SLAM) sensors, including cameras and depth sensors, to scan and map the user's physical environment. This tracking allows virtual objects to remain stable relative to the real-world setting. Additionally, many AR headsets employ bone-conduction speakers or open-air audio systems that deliver sound without isolating the user from real-world audio cues. In terms of control, AR devices may incorporate gesture-tracking sensors, allowing users to interact with virtual objects through hand and finger movements. Processing power for AR applications is generally managed within the headset itself, though some devices may require external computing support for more complex tasks.

Furthermore, Extended Reality (XR) headsets represent a broad category that encompasses the full spectrum of immersive technologies, including elements of both VR and AR, and often add enhanced functionalities for a more versatile user experience. XR headsets aim to merge the immersive capabilities of VR with the interactive, real-world integration of AR, allowing users to switch seamlessly between fully virtual environments, augmented overlays, and combinations of both. These devices often may include high-resolution displays with adaptive optics that can adjust the transparency or opacity of the visuals, enabling a smooth transition from complete immersion in virtual content to partially transparent overlays on the real world. With powerful onboard processors or wireless connections to external computers, XR headsets handle complex computations needed for fluid transitions between virtual and augmented experiences, making them suitable for a wide array of applications, from professional training and simulation to entertainment and remote collaboration.

So-called Smart glasses, on the other hand, provide a more lightweight experience, offering heads-up display (HUD) capabilities for simpler applications like notifications, navigation, and hands-free interaction. Unlike VR/AR and XR headsets, smart glasses do not focus on immersion and often have lower display resolutions, using compact display modules like Micro-LEDs or monochrome OLEDs. They rely on simple optics, such as prisms or waveguides, to project basic visual information onto the user's field of vision. Because smart glasses are designed primarily for low-power, everyday use, they typically lack the environment-mapping and SLAM sensors of AR headsets. However, they may still include very basic motion sensors like accelerometers and gyroscopes to support simple tracking functions. For interaction, many smart glasses utilize voice commands or touchsensitive controls on the frame. The onboard processor is generally optimized for energy efficiency to extend battery life, supporting low-power functionalities for daily use.

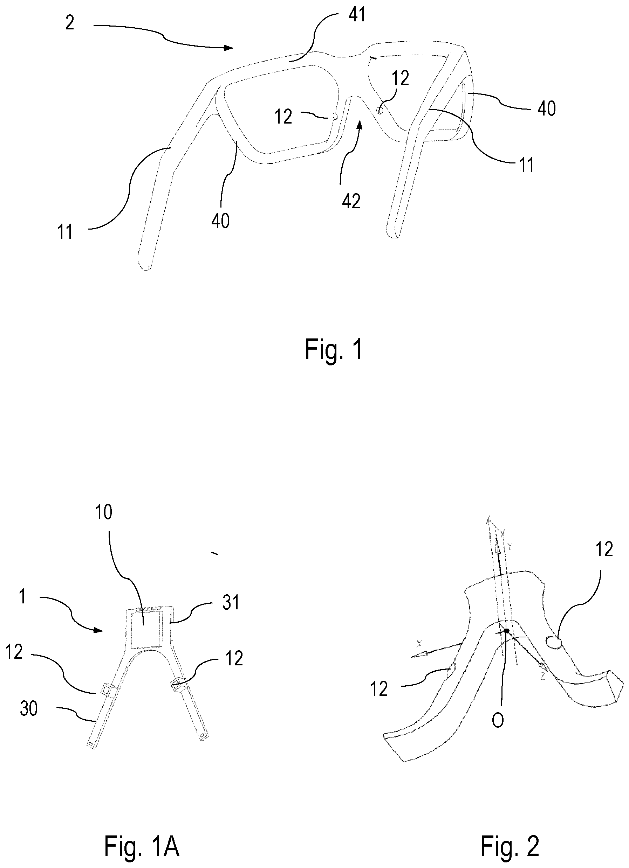

From now on, a head-mounted device 2 is intended for instance VR/AR/XR headsets, so-called smart glasses or any electronic device embedding or connectable to the eye descriptor unit 1 (fig. 1A).

For the sake of clarity of the present specification, a horizontal plane is meant to be a plane that is perpendicular to the local direction of gravity.

Furthermore, throughout this specification, the acronym "PCB" is understood to mean a printed circuit board.

In this specification, reference is being made to the reference system shown in figure 2.

The coordinate system of the reference system is selected such that the symmetry plane of the invented head-mounted device 2 is the plane YZ, spanned by the Y axis and the Z axis. The XZ plane (which may be a horizontal plane), is spanned by the Z axis and the X axis on the right and left sides of the head-mounted device 2, each intersecting opposing and symmetrically arranged regions divided by the symmetry plane YZ. Assuming the reference system (X, Y, Z) be placed with its origin corresponding to the nose bridge point O, which is the midpoint of the lower edge of the nose bridge with respect to the cross-section on the symmetry plane of the head-mounted device 2, and the Z axis is parallel to the ZX plane and the Y axis is directed upward, as shown in figure 2.

With respect to different configurations of the temples 11 of various head-mounted devices 2, the XZ plane is defined as the plane passing through the noise point O defined above and the temple points E100, .... , E125, defined as the points on the lower edge of the temple 11, that is assumed to be in contact with the top of the ear when in use, said temple points E100, .... , E125 which are at any one of the following distances 100 mm or 105 mm or 110 mm or 115 mm or 120 mm or 125 mm, or being any of the points having distance from the nose bridge point O in the range 100 mm to 125 mm, measured from the nose bridge point O on the Z axis, based on different form-factor of the head-mounted devices and head sizes and shapes of users

The right eye sensor and the left eye sensor 12 are each arranged tilted with respect to these planes or axes. According to one preferred embodiment, it is provided that the optical axis of the eye sensor 12 is arranged with a yaw angle α, in relation to the Z axis measured in the plane XZ, and the yaw angle α is between 20 and 50 degrees ±2 degrees as manufacturing and assembly tolerance, said range defined by absolute values of α with respect to the Z axis in the ZX plane. In addition, it is provided according to a further preferred embodiment that one optical axis of the two eye sensors 12 is arranged at a yaw angle α in relation to the Z axis, as an absolute value, and the yaw angle α is between 25° and 35°.

Furthermore, it is provided according to a preferred embodiment compatible with all the embodiments described in the present specification, that the optical axis of the two eye sensors 12 is arranged at a pitch angle β, with respect to the Z axis measured in the YZ plane as an absolute value, and the pitch angle β is in the range of 0 to 40 degrees, preferably in the range of 0 to 20 degrees, ±2 degrees as manufacturing and assembly tolerance, said range defined by absolute values of β with respect to the Z axis in the ZY plane.

The pitch angle β optimizes the different configurations of head-mounted devices 2 according to different head sizes and shapes of users, as well as different glasses form-factors (regular eyeglasses or more curved/sporty spectacles/head-mounted devices), while the yaw angle α addresses different head sizes of users, having regard to the horizontal extension of the user's head.

**In** a further embodiment, compatible with all the embodiments described in the resent specification, to avoid the risk of having eyelids/eyelashes/cheekbones interferences considering also different nose shapes of different anatomical traits of different ethnic groups in combination with different form-factors of the head-mounted devices 2 embedding an eye-descriptor unit 1, the eye sensor 12 are arranged in the corresponding nose part frame 42 in a position referred to a Y axis of the reference system in the range in the range Ŷ - 5 mm ≤ Y ≤ Ŷ + 5 mm, where Y < 0 and where -Y = δ + λ * tan β, with δ = 3.4 mm and λ = 19.5 mm according to measurements taken for a population of people from different ethnic groups including Asian and European people, and considering different shapes/sizes and form-factors of the head-mounted device 2 in use.

The range defined above has also been optimized considering the space needed for the electronic components in the nose bridge area 42 of the eye descriptor unit 1 described below.

The eye descriptor unit 1, according to an embodiment of the present invention, is a hardware structure which is shaped to be integrated into a wearable U-shaped nose bridge portion compatible with the nose of a user, thus being configured to make the head-mounted device 2 easily customizable for different nose shapes when the eye descriptor unit 1 is embedded into said head-mounted device 2. The wearable U-shaped nose bridge portion may be in the form of an eye-tracking module applicable to eyeglasses or smart glasses, or VR/XR head-mounted devices in general.

Being the eye sensors 12 configured to face the user's eye, in a preferred solution the eye sensor 12 are arranged in a first U-shaped PCB nose-bridge part 30 and the edge computing unit 10 in a second PCB part 31.

According to an embodiment, compatible with all the possible different embodiments of the present specification, the second PCB part 31 may be an extension of the first U-shaped PCB nose-bridge part 30, which is shaped to be integrated into a Wearable U-shaped nose bridge portion compatible with the nose of a wearer when in use.

According to a further embodiment, compatible with all the possible different embodiments of the present specification, the second PCB part 31 is electronically connected to the first U-shaped PCB nose-bridge part 30, but it may be arranged in a different area other than a wearable U-shaped nose bridge portion compatible with the nose of a wearer when in use.

To avoid any interference with the field of view of the user, the footprint of the eye-descriptor unit 1 and of the eye sensor 12 is extremely reduced, wherein the wearable U-shaped nose bridge portion of the head-mounted device is designed in such a way that the eye sensor 12 is integrated in the arms of the nose bridge portion, without causing any protruding part or localized swelling of the frame embedding the eye sensor 12, possibly interfering with the field of view of the user or with the wearing comfort of the head-mounted device in use.

According to a further preferred solution, the head-mounted device 2 comprises an upper part frame, a wearable U-shaped nose bridge portion 42 connected to the center of the upper part frame 41, a left and right side part frames 40, wherein the combination of said parts of the frame 40 is configured to hold in place lenses and to make the lenses replaceable by retaining means. Said retaining means may be configured as snap-fit mechanisms, for example. This feature can address the possibility of using the head-mounted devices by user having vision disorders.

To improve the head-mounted device 2 assembly process, in particular having regard to the integration of the eye-descriptor unit 1, the nose bridge frame part 42 is preferably formed by at least two main parts, those being a carrier and a cover, wherein both said carrier and said cover are each other a single unitary structure manufactured by an additively manufacturing process. The additive manufacturing process allows more flexibility in creating a customized shape of the nose bridge part of the head-mounted device 2, according to the needs of the eye-descriptor unit 1, keeping low manufacturing costs.

As anticipated, the eye descriptor unit 1 is arranged in the nose bridge portion of the head-mounted device 2, and to keep valid extrinsic calibrations set up of the head-mounted device 2, its nose bridge area must be robust enough and stable during usage. Therefore, it's preferable that the carrier is made of a solid additive material from which is subtracted just the space for the eye descriptor unit 1 and its electronic connections with the head-mounted device 2.

With particular regard to the eye descriptor unit 1, is designed according to the edge computing principles, i.e. the computation of the data occurs where or close to where the input data are generated. The eye sensors 12 generate the eye representation data, which are then processed as an array of the user's eye-descriptors directly by the edge computation unit 10 in the eye descriptor unit 1.

The eye descriptor unit 1 (fig. 1A) is provided with at least one eye sensor 12 facing the user's left eye and one eye sensor 12 facing the user's right eye and an edge computation unit 10 provided with a machine learning hardware accelerator configured to receive as input user's eye representation data from the eye sensors 12 and to send as output an array of user's eye-descriptors when in use and wherein the edge computation unit 10 is programmed with a customized bare metal code or with Real-Time Operating System RTOS for microcontrollers or with a combination of customized bare metal code and Real-Time Operating System RTOS for microcontrollers.

The output, being the array of user's eye descriptor, is provided with reference to the extrinsic parameters (rotations about the axes x, y, z of the original coordinate system of the eye descriptor unit 1) of the eye descriptor unit 1.

The output, being the array of user's eye descriptor, may include x, y, z-components of the 3D user's gaze directions based on the extrinsic parameters of the eye descriptor unit 1 once embedded in the head-mounted device 2 and according to further possible embodiments, it may include other eye geometry parameters (eyeball center 3D coordinates, pupil center 3D coordinates), and/or pupil size data, and/or blink state, and/or vergence angle.

To provide a full characterization of the user's eye, the array of eye descriptors are selected from a list consisting of: a pupil plane, a 2D pupil center, a confidence value for the 2D pupil center, a major radius of the 2D pupil ellipse, a confidence value for the major radius of the 2D pupil ellipse, a minor radius of the 2D pupil ellipse, a confidence value for the minor radius of the 2D pupil ellipse, an orientation of the 2D pupil ellipse, a confidence value for the orientation of the 2D pupil ellipse, a pupil center ray, a projected pupil center ray, a 3D extrusion of the projected pupil center ray, a depth plane, an eyeball center plane, a 3D eyeball center, a confidence value for the 3D eyeball center, a 3D pupil center, a confidence value for the 3D pupil center, a distance between the eyeball center and an eye camera, an eyeball radius, an optical axis, a visual axis, a line of sight, a 3D gaze direction, a monocular 3D gaze direction, a confidence value for the monocular 3D gaze direction, a vergence angle, a binocular 3D gaze point, a confidence value for the binocular 3D gaze point, a binocular 2D gaze point, a confident value for the binocular 2D gaze point, a pupil diameter, a confidence value for the pupil diameter, an apparent pupil diameter, a pupil area, an iris diameter, a confidence value for the iris diameter, a corneal limbus, a major radius of the corneal limbus, a confidence value for the major radius of the corneal limbus, a minor radius of the corneal limbus, a confidence value for the minor radius of the corneal limbus, an orientation of the corneal limbus, a confidence value for the orientation of the corneal limbus, a distance between the corneal limbus center and the pupil center, a curvature of the corneal front surface expressed in keratometric diopters, a distance between the corneal apex and the pupil center, a distance between the corneal vertex and the pupil center, an index of refraction of the cornea, an index of refraction of the anterior chamber of the eye, an index of refraction of the posterior chamber of the eye, an index of refraction of the crystalline lens, a distance between the crystalline lens and the 3D eyeball center, a sclera with certain light scattering properties, an eye open/closed state classification, an angular speed of the eyeball, an angular acceleration of the eyeball, eye movements statistics, a classification of the binocular gaze into blink/fixation/pursuit/saccade/vestibulo-ocular reflex/microssacade events, statistics for the evaluation of the cognitive load of the user, statistics for the evaluation of the attention of the user, statistics for the evaluation of the awareness of the user, user's eye features and eye statistics for user identification and/or authentication.

Some or preferably all of the eye descriptors mentioned above are calculated in real time, when the inference time of the user's eye features array is smaller than the acquisition time of the user's eye representation data from the eye sensor 12.

With regard to the edge computation unit 10, one preferred solution is that it is a microcontroller programmed with a customized bare-metal code or with Real-Time Operating System RTOS for microcontrollers or with a combination of customized bare-metal code and Real-Time Operating System RTOS for microcontrollers, in order to achieve very low power consumption.

Bare-metal code is defined as software that runs directly on hardware without the intermediation of an operating system or virtualization layer, thereby bypassing all abstraction layers associated with standard platforms, kernels, or system calls. This code interacts with the system's resources at the lowest possible level, typically involving direct manipulation of hardware registers and immediate access to peripherals. Bare-metal code characterizes executable instructions hard-coded to the specific configuration of hardware.

**In** contrast, general-purpose operating systems (GPOS), such as Windows, Linux, and MacOS, typically introduce performance overhead due to their kernel operations, which can result in jitter and delays in response times. By facilitating direct hardware access, bare-metal systems enhance the ability to maintain precise timing and deterministic behavior, thereby ensuring minimal latency in system operations.

It may be useful to clarify that a controller is an extremely general term to define a device or system component designed to manage, regulate, or direct the operation of other devices or systems. Controllers execute commands based on inputs they receive, process these commands through programmed logic or algorithms, and produce outputs that adjust or control certain behaviors within a system. They can vary in complexity, from simple analog controllers that respond to electrical signals to complex digital controllers that execute software-based instructions. Controller may be: PLC, Microprocessor, Microcontrollers, FPGA (like for instance ICE40 from Lattice Semiconductors), Motor controller.

Furthermore, it's necessary to distinguish between microprocessor and microcontrollers. According to an article written on 13/6/2024 by Josh Schneider on the IBM website (https://www.ibm.com/think/topics/microcontroller-vs-microprocessor):
*"Microcontroller units (MCUs) and microprocessor units (MPUs) are two kinds of integrated circuits that, while similar in certain ways, are very different in many others. Replacing antiquated multi-component central processing units (CPUs) with separate logic units, these single-chip processors are both extremely valuable in the continued development of computing technology. However, microcontrollers and microprocessors differ significantly in component structure, chip architecture, performance capabilities and application.*
*The key difference between these two units is that microcontrollers combine all the necessary elements of a microcomputer system onto a single piece of hardware. Microcontrollers do not require additional peripherals or complex operating systems to function, while microprocessors do. Both circuits contain CPUs, however, microcontrollers also integrate memory, input*/*output (I*/*O) components and other varied peripherals.*
*Cost-effective and small-in-size, low-power microcontrollers are optimized for all-in-one functionality. As a result, these units are best used for specific applications like automotive infotainment systems and Internet-of- Things (IoT) devices."*

Regarding the hardware configuration, John Schneider explains and distinguishes:
*"Key components of a microcontroller*
   - *Central processing unit (CPU): Colloquially referred to as the computer's "brain," the CPU is responsible for executing instructions and controlling operations.*
   - *Memory: Microcontrollers contain both volatile memory (RAM), which stores temporary data that may be lost if the system loses power, and non-volatile flash memory (ROM) for storing the microcontroller's programming code.*
   - *Peripherals: Depending on the intended application, a microcontroller may contain various peripheral components, such as I*/*O interfaces, timers, counters, analog-to-digital converters (ADCs) and communication protocols (UART, SPI, 12C).*
   *(...)*
*Key components of a microprocessor*
   *Modern microprocessors combine millions of small transistors, resistors and diodes assembled on a semiconductor material to create the key components of a CPU.*
   - *Arithmetic logic unit (ALU): The main logic unit of the CPU, this component executes logical operations including mathematical calculations and data comparisons.*
   - *Control unit (CU): The CU circuit interprets instructions and initiates their execution, directing the basic operations of the processor.*
   - *Registers: Small, fast memory storage used by a CPU to temporarily hold data and instructions during computational processes.*
   - *Cache memory: Microprocessors and CPUs use cache memory, a high-speed form of memory located close to the CPU, to store frequently accessed data to accelerate performance.*
   - *Processor cores: Individual processing units within microprocessors are known as cores. Modern processors frequently incorporate multiple cores (dual-core, quad-core) allowing for parallel processing by enabling the performance of multiple tasks simultaneously.*
   - *I*/*O modules: A microprocessor's I*/*O components are critical for managing the flow of data to and from the CPU, including any additional computer peripherals including networking peripherals such as ethernet ports or WiFi units."*

Therefore, a microcontroller (MCU) is a compact, integrated computing device designed for deterministic control of hardware systems. In essence, it is a very specific controller hardware architecture. It operates either without an operating system, known as "bare metal," or with a "Real-Time Operating System (RTOS)", or with a combination of customized bare metal code and Real-Time Operating System RTOS for microcontrollers, providing predictable, time-bound task execution essential for real-time applications and very power efficient, and both of them may be used in the eye descriptor unit 1 of the present invention. Unlike general-purpose processors (like for instance CortexA architecture as Cortex-A53), microcontrollers do not support general-purpose operating systems (e.g., Windows or Linux) due to their focus on efficient, low-level control and minimal latency requirements. Therefore, describing a microcontroller as a general-purpose processor is not correct, because it recalls the need to implement a general-purpose operating system, which a microcontroller does not have the capability to do.

Microcontrollers are also optimized for low-power operation, with the capability to achieve sub-millisecond wake-up times from sleep modes. This feature allows a microcontroller-based system to enter low-power states between processing intervals or frames, making it suitable for applications with strict power management requirements.

A microcontroller relies on deterministic interrupting handling systems, which ensures that they can respond rapidly to hardware events, typically within a few microseconds. Microcontrollers are designed to prioritize interruptions with minimal latency, allowing them to address high-priority events in real-time. **In** general-purpose systems, interrupting latency is often less predictable due to the overhead of multitasking operating systems and other background processes.

The interrupt handling, plus a bare metal or RTOS or both allows for a deterministic system, avoiding most of the problems of synchronization.

The left and right eye sensors 12 may be camera-based sensors but may be different kinds of sensors like lasers able to scan user's eyes to detect position of pupils or micro electromechanical systems like for instance MEMS-based scanning elements.

When the eye sensors 12 are camera-based sensors, they may be monochromatic cameras, or DVS for example or other types of sensors. In that case, the eye representations are eye images where at least the user's pupil is included.

When the eye sensors 12 are camera-based sensors the eye descriptor unit 1 may include optics, allowing to control the amount of light that enters the cameras.

The eye descriptor unit 1 may include Infrared LED. Infrared (IR) LEDs are components in eye-tracking devices, primarily because they provide a source of invisible light that illuminates the eyes without causing distraction, as IR light lies outside the visible spectrum. This illumination helps to capture clear images of the eyes, even in low-light conditions.

The IR light may also enhance contrast for key eye features, such as the pupil and iris. It also creates a bright reflection, known as the "glint," by reflecting off the retina, which helps distinguish the pupil from other parts of the eye.

The eye descriptor unit 1 may include a front camera (not visible in the drawings), making this technical aspect compatible with all the possible embodiments disclosed in the present specification.

While the eye sensors 12 are facing the eyes of the user, the front camera is configured to capture images corresponding to a field of view possibly perceived by the user in front of him/her, when wearing the head-mounted device 2 equipped with the eye-tracking unit 1. Such images of the user's field of view are hereinafter designated as scene images. The front camera is therefore not intended for imaging the user's eye.

In each embodiment disclosed in the present specification the edge computation unit 10, the memory and the data interface may be connected at least indirectly to an energy accumulator by circuitry.

In each embodiment disclosed in the present specification the output, being an array of user's eye descriptors, may be a text-based file.

In each embodiment disclosed in the present specification, the eye descriptor unit 1 may have a maximum power consumption of 200 mW.

The present invention furthermore relates to a method for detecting user's eye descriptors using a head-mounted device 2, embedding an eye descriptor unit 1, worn by a user, comprising the following steps:
- receive as input at least two user's eye representations data at two different time instances from the eye sensors,
- estimate the user's eye geometry based on the two user's eye representations, and
- send as output the array of eye descriptors of the user.

**In** a further embodiment compatible with all possible variations described in the present specification, at least one eye descriptor is inferred by at least one neural network step.

According to a further preferable embodiment, the at least one neural network step implements a neural network architecture for localizing at least one user's eye features in the user's eye representation input, wherein the neural network step input and/or neural network step outputs, and/or the neural network step weights are quantized to 8-bit or fewer and/or the neural network step activations are quantized to 16-bit or fewer.

Quantization is a technique that reduces computational power consumption by decreasing the precision of the numerical values used to represent a model's weights and activations. Typically, neural networks use 32-bit floating-point numbers for these computations. Quantization converts these to lower precision formats, such as 8-bit integers. This reduction in precision means that the neural network model's parameters in the eye-tracking pipeline and calculations require less memory and bandwidth during transmission inside hardware.

Lower-precision integer arithmetic is much less demanding from a computational perspective compared to floating-point arithmetic. In addition, quantized models take up less space in memory, which means data can be accessed and moved more efficiently-a major contributor to overall power savings. Memory access is typically a significant part of the energy budget in neural network inference in eye-tracking inference process, so shrinking the data size with quantization leads to further energy savings.

Quantization may also enable better use of specialized low-power hardware as edge computing units, such as machine-learning accelerators and microcontrollers that are optimized for integer math. In fact, a further preferred embodiment of the power-efficient method is configured to cause, during operations, the edge computation unit 10 electronically connected to the eye sensor 12 to use a maximum of 200 mW, preferably a maximum of 100 mW, most preferably a maximum of 50 mW of power, wherein the edge computation unit 10 is a microcontroller according to the definitions mentioned in the present specification.

## Claims

1. A head-mounted device (2), implementing an eye descriptor unit (1) for estimating the user's gaze once the eye descriptor unit (1) is connected to the head-mounted device (2) worn by a user when in use, said eye descriptor unit (1) comprising a first U-shaped PCB nose bridge part (30) which is shaped for being integrated into a wearable U-shaped nose bridge portion of the head-mounted device (2) compatible with the nose of a wearer, said first U-shaped PCB nose bridge portion (30) including at least one eye sensor (12) facing the user's left eye and one eye sensor (12) facing the user's right eye, said eye descriptor unit (1) comprising a second PCB part (31) including an edge computation unit (10) electronically connected to the eye sensors (12) by an electronic circuitry, said edge computation unit (10) provided with a machine learning hardware accelerator and configured to receive as input user's eye representation data from the eye sensors (12) and to send as output an array of user's eye-descriptors when in use and wherein the edge computation unit (10) is a microcontroller programmed with a customized bare metal code or with Real-Time Operating System RTOS for microcontrollers or with a combination of customized bare metal code and Real-Time Operating System RTOS for microcontrollers.

2. The head-mounted device (2) according to claim 1, wherein the wearable U-shaped nose bridge portion of the head-mounted device is designed in such a way that the eye sensor (12) is integrated in the arms of the nose bridge portion (42), without causing any protruding part of the frame embedding the eye sensor (12) interfering with the field of view of the user when in use.

3. The head-mounted device (2) according to any of the preceding claims, wherein an angle β is in the range 0 degrees ≤ β ≤ 40 degrees, preferably in the range 0 degrees ≤ β ≤ 20 degrees ± 2 degrees as manufacturing and assembly tolerance, said range defined by absolute values of β with respect to the Z axis, said angle β projected on the plane YZ which is the symmetry plane of the head-mounted device (2), assuming a reference system (X, Y, Z) being placed with its origin corresponding to the nose bridge point (O), the XZ plane of the reference system passing through the nose point (O), defined as the highest midpoint of the lower edge of the nose bridge with respect to the cross-section on the symmetry plane of the head-mounted device (2), and the temple points (E100, ... , E125), defined as the point on the lower edge of the temples (11) that is assumed to be in contact with the top of the ear when in use, said temple points (E100, ... , E125) which are at any of the following distances 100 mm or 105 mm or 110 mm or 115 mm or 120 mm or 125 mm, or being any of the points having distance from the nose bridge point (O) in the range 100 mm to 125 mm, measured from the nose bridge point (O) on the Z axis.

4. The head-mounted device (2) according to claim 3, wherein the left eye sensor (12) and the right eye sensor (12) are arranged in the corresponding nose part frame (42) in a position with a Y coordinate referred to the Y axis of the reference system in the range Y - 5 mm ≤ Y ≤ Ŷ + 5 mm, where Y < 0 and where - Y = δ + λ * tan β, with δ = 3.4 mm and λ = 19.5 mm.

5. The head-mounted device (2) according to any of the preceding claims, wherein an angle α is in the range 20 degrees ≤ α ≤ 50 degrees ± 2 degrees as manufacturing and assembly tolerance, said range defined by absolute values with respect to the Z axis in the ZX plane, said angle α lying on the ZX plane passing through the nose bridge point (O), defined as the highest midpoint of the lower edge of the nose bridge with respect to the cross-section on the symmetry plane of the head-mounted device (2), and the temples points (E100, ... , E125), defined as the point on the lower edge of the temple (11) that is assumed to be in contact with the top of the ear when in use, said temple points (E100, ... , E125) which are at anyone of the following distances 100 mm or 105 mm or 110 mm or 115 mm or 120 mm or 125 mm, or being any of the points having distance from the nose bridge point (O) in the range 100 mm to 125 mm, measured from the nose bridge point (O) on the Z axis.

6. The head-mounted device (2) according to any of the preceding claims, wherein the frame of the head-mounted device (2) comprises an upper part frame (41), a wearable U-shaped nose bridge portion (42) connected to the center of the upper part frame (41), a left and right side part frames (40), wherein the combination of said parts of the frame (40) is configured to hold in place lenses and to make the lenses replaceable when they are arranged on the head-mounted-device (2).

7. The head-mounted device (2) according to any of the preceding claims, wherein the eye descriptor unit (1) comprises Infrared LED arranged in the first U-shaped PCB nose bridge part (30).

8. The head-mounted device (2) according to any of the preceding claims, wherein the eye descriptor unit (1) comprises a front camera to capture the field of view of the user when in use.

9. The head-mounted device (2) according to any of the preceding claims, wherein the nose bridge frame (42) is formed by at least two main parts, those being a carrier and a cover, wherein both said carrier and said cover are each other a single unitary structure manufactured by an additive manufacturing process.

10. The head-mounted device (2) according to claim 9, wherein the carrier is made of a solid additive material from which is subtracted just the space for the eye descriptor unit (1) and its electronic connections with the head-mounted device (2).

11. A method for determining an array of gaze data, wherein the method is performed using the head-mounted device (2) according to any of the preceding claims, wherein the head-mounted device (2) is configured to:
- receive as input at least two user's eye representation data at two different time instances from the eye sensors (12),
- estimate the user's eye geometry based on the input, and
- send as output the array of eye descriptors of the user.

12. The method according to claim 11, wherein the inference time of the user's eye features array is smaller than the acquisition time of the user's eye representation data from the eye sensor (12), allowing the method to provide real-time user's eye features.

13. The method according to any of the preceding claims 11 to 12, wherein the array of eye descriptors are selected from a list consisting of: a pupil plane, a 2D pupil center, a confidence value for the 2D pupil center, a major radius of the 2D pupil ellipse, a confidence value for the major radius of the 2D pupil ellipse, a minor radius of the 2D pupil ellipse, a confidence value for the minor radius of the 2D pupil ellipse, an orientation of the 2D pupil ellipse, a confidence value for the orientation of the 2D pupil ellipse, a pupil center ray, a projected pupil center ray, a 3D extrusion of the projected pupil center ray, a depth plane, an eyeball center plane, a 3D eyeball center, a confidence value for the 3D eyeball center, a 3D pupil center, a confidence value for the 3D pupil center, a distance between the eyeball center and an eye camera, an eyeball radius, an optical axis, a visual axis, a line of sight, a 3D gaze direction, a monocular 3D gaze direction, a confidence value for the monocular 3D gaze direction, a vergence angle, a binocular 3D gaze point, a confidence value for the binocular 3D gaze point, a binocular 2D gaze point, a confident value for the binocular 2D gaze point, a pupil diameter, a confidence value for the pupil diameter, an apparent pupil diameter, a pupil area, an iris diameter, a confidence value for the iris diameter, a corneal limbus, a major radius of the corneal limbus, a confidence value for the major radius of the corneal limbus, a minor radius of the corneal limbus, a confidence value for the minor radius of the corneal limbus, an orientation of the corneal limbus, a confidence value for the orientation of the corneal limbus, a distance between the corneal limbus center and the pupil center, a curvature of the corneal front surface expressed in keratometric diopters, a distance between the corneal apex and the pupil center, a distance between the corneal vertex and the pupil center, an index of refraction of the cornea, an index of refraction of the anterior chamber of the eye, an index of refraction of the posterior chamber of the eye, an index of refraction of the crystalline lens, a distance between the crystalline lens and the 3D eyeball center, a sclera with certain light scattering properties, an eye open/closed state classification, an angular speed of the eyeball, an angular acceleration of the eyeball, eye movements statistics, a classification of the binocular gaze into blink/fixation/pursuit/saccade/vestibulo-ocular reflex/microssacade events, statistics for the evaluation of the cognitive load of the user, statistics for the evaluation of the attention of the user, statistics for the evaluation of the awareness of the user, user's eye features and eye statistics for user identification and/or authentication.

14. The method according to any of the preceding claims 11 to 13, wherein at least one eye descriptor is inferred by at least one neural network step.

15. The method according to claim 14, wherein the at least one neural network step implements a neural network architecture for localizing at least one user's eye features in the user's eye representation input, wherein the neural network step input and/or neural network step outputs, and/or the neural network step weights are quantized to 8-bit or fewer and/or the neural network step activations are quantized to 16-bit or fewer.

## Patentansprüche

1. Am Kopf getragene Vorrichtung (2), die eine Augendeskriptoreinheit (1) implementiert, um den Blick des Benutzers zu schätzen, sobald die Augendeskriptoreinheit (1) mit der beim Gebrauch von einem Benutzer getragenen am Kopf getragenen Vorrichtung (2) verbunden ist, wobei die Augendeskriptoreinheit (1) einen ersten U-förmigen Leiterplatten-Nasenbrückenteil (30) umfasst, der so ausgebildet ist, dass er in einen tragbaren U-förmigen Nasenbrückenabschnitt der am Kopf getragenen Vorrichtung (2), der mit der Nase eines Trägers kompatibel ist, integriert werden kann, wobei der erste U-förmige Leiterplatten-Nasenbrückenabschnitt (30) mindestens einen auf das linke Auge des Benutzers gerichteten Augensensor (12) und einen auf das rechte Auge des Benutzers gerichteten Augensensor (12) umfasst, wobei die Augendeskriptoreinheit (1) einen zweiten Leiterplattenteil (31) umfasst, der eine über eine elektronische Schaltung elektronisch mit den Augensensoren (12) verbundene Edge-Recheneinheit (10) enthält, wobei die Edge-Recheneinheit (10) mit einem Hardwarebeschleuniger für maschinelles Lernen versehen und dazu konfiguriert ist, als Eingabe Augendarstellungsdaten des Benutzers von den Augensensoren (12) zu empfangen und als Ausgabe im Gebrauch ein Array von Augendeskriptoren des Benutzers auszugeben, und wobei die Edge-Recheneinheit (10) ein Mikrocontroller ist, der mit einem angepassten Bare-Metal-Code oder mit einem Echtzeitbetriebssystem RTOS für Mikrocontroller oder mit einer Kombination aus angepasstem Bare-Metal-Code und einem Echtzeitbetriebssystem RTOS für Mikrocontroller programmiert ist.

2. Am Kopf getragene Vorrichtung (2) nach Anspruch 1, wobei der tragbare U-förmige Nasenbrückenabschnitt der am Kopf getragenen Vorrichtung so ausgelegt ist, dass der Augensensor (12) in die Arme des Nasenbrückenabschnitts (42) integriert ist, ohne dass irgendein hervorstehender Teil des den Augensensor (12) aufnehmenden Rahmens das Sichtfeld des Benutzers beim Gebrauch beeinträchtigt.

3. Am Kopf getragene Vorrichtung (2) nach einem der vorhergehenden Ansprüche, wobei ein Winkel β im Bereich von 0 Grad ≤ β ≤ 40 Grad, vorzugsweise im Bereich von 0 Grad ≤ β ≤ 20 Grad ± 2 Grad als Fertigungs- und Montagetoleranz, liegt, wobei dieser Bereich durch die Absolutwerte von β bezüglich der Z-Achse definiert ist, wobei der Winkel β auf die YZ-Ebene projiziert wird, die die Symmetrieebene der am Kopf getragenen Vorrichtung (2) ist, unter Annahme eines Bezugssystems (X, Y, Z), dessen Ursprung dem Nasenbrückenpunkt (O) entspricht, wobei die XZ-Ebene des Bezugssystems durch den Nasenpunkt (O) verläuft, der als höchster Mittelpunkt der unteren Kante der Nasenbrücke bezüglich des Querschnitts in der Symmetrieebene der am Kopf getragenen Vorrichtung (2) definiert ist, sowie durch die Bügelpunkte (E100, ... , E125), die als derjenige Punkt an der unteren Kante der Bügel (11) definiert sind, der beim Gebrauch als mit dem oberen Teil des Ohrs in Kontakt stehend angenommen wird, wobei die Bügelpunkte (E100, ... , E125) in einem Abstand von 100 mm oder 105 mm oder 110 mm oder 115 mm oder 120 mm oder 125 mm liegen oder beliebige Punkte mit einem Abstand vom Nasenbrückenpunkt (O) im Bereich von 100 mm bis 125 mm sind, gemessen vom Nasenbrückenpunkt (O) entlang der Z-Achse.

4. Am Kopf getragene Vorrichtung (2) nach Anspruch 3, wobei der linke Augensensor (12) und der rechte Augensensor (12) in dem entsprechenden Nasenteilrahmen (42) an einer Position mit einer Y-Koordinate bezüglich der Y-Achse des Bezugssystems im Bereich Ŷ - 5 mm ≤ Y ≤ Ŷ + 5 m angeordnet ist, wobei Y < 0 und wobei - Ŷ = δ + λ * tan β, mit δ = 3.4 mm und λ = 19.5 mm gilt.

5. Am Kopf getragene Vorrichtung (2) nach einem der vorhergehenden Ansprüche, wobei ein Winkel α im Bereich von 20 Grad ≤ α ≤ 50 Grad ± 2 Grad als Fertigungs- und Montagetoleranz liegt, wobei dieser Bereich durch Absolutwerte bezüglich der Z-Achse in der ZX-Ebene definiert ist, wobei der Winkel α in der ZX-Ebene liegt, die durch den Nasenbrückenpunkt (O) verläuft, der als höchster Mittelpunkt der unteren Kante der Nasenbrücke bezüglich des Querschnitts in der Symmetrieebene der am Kopf getragenen Vorrichtung (2) definiert ist, sowie durch die Bügelpunkte (E100, ... , E125), die als derjenige Punkt an der unteren Kante des Bügels (11) definiert sind, der beim Gebrauch als mit dem oberen Teil des Ohrs in Kontakt stehend angenommen wird, wobei die Bügelpunkte (E100, ... , E125) in einem Abstand von 100 mm oder 105 mm oder 110 mm oder 115 mm oder 120 mm oder 125 mm liegen oder beliebige Punkte mit einem Abstand vom Nasenbrückenpunkt (O) im Bereich von 100 mm bis 125 mm sind, gemessen vom Nasenbrückenpunkt (O) entlang der Z-Achse.

6. Am Kopf getragene Vorrichtung (2) nach einem der vorhergehenden Ansprüche, wobei der Rahmen der am Kopf getragenen Vorrichtung (2) einen oberen Rahmenteil (41), einen tragbaren U-förmigen Nasenbrückenabschnitt (42), der mit dem Zentrum des oberen Rahmenteils (41) verbunden ist, sowie einen linken und einen rechten Seitenrahmenteil (40) umfasst, wobei die Kombination dieser Rahmenteile (40) dazu konfiguriert ist, Gläser an Ort und Stelle zu halten und die Gläser austauschbar zu machen, wenn sie an der am Kopf getragenen Vorrichtung (2) angeordnet sind.

7. Am Kopf getragene Vorrichtung (2) nach einem der vorhergehenden Ansprüche, wobei die Augendeskriptoreinheit (1) eine im ersten U-förmigen Leiterplatten-Nasenbrückenteil (30) angeordnete Infrarot-LED umfasst.

8. Am Kopf getragene Vorrichtung (2) nach einem der vorhergehenden Ansprüche, wobei die Augendeskriptoreinheit (1) eine Frontkamera umfasst, um beim Gebrauch das Sichtfeld des Benutzers zu erfassen.

9. Am Kopf getragene Vorrichtung (2) nach einem der vorhergehenden Ansprüche, wobei der Nasenbrückenrahmen (42) durch mindestens zwei Hauptteile gebildet ist, nämlich einen Träger und eine Abdeckung, wobei sowohl der Träger als auch die Abdeckung jeweils eine einstückige Struktur sind, die durch ein additives Fertigungsverfahren hergestellt ist.

10. Am Kopf getragene Vorrichtung (2) nach Anspruch 9, wobei der Träger aus einem massiven additiven Material besteht, von dem lediglich der Raum für die Augendeskriptoreinheit (1) und deren elektrische Verbindungen mit der am Kopf getragenen Vorrichtung (2) ausgespart ist.

11. Verfahren zum Bestimmen eines Arrays von Blickdaten, wobei das Verfahren unter Verwendung der am Kopf getragenen Vorrichtung (2) nach einem der vorhergehenden Ansprüche durchgeführt wird, wobei die am Kopf getragene Vorrichtung (2) dazu konfiguriert ist:
- mindestens zwei Augendarstellungsdaten des Benutzers zu zwei unterschiedlichen Zeitpunkten von den Augensensoren (12) als Eingabe zu empfangen,
- auf Grundlage der Eingabe die Augengeometrie des Benutzers zu schätzen, und
- als Ausgabe das Array von Augendeskriptoren des Benutzers auszugeben.

12. Verfahren nach Anspruch 11, wobei die Inferenzzeit des Arrays von Augenmerkmalen des Benutzers kleiner ist als die Erfassungszeit der Augendarstellungsdaten des Benutzers vom Augensensor (12), sodass das Verfahren Augenmerkmale des Benutzers in Echtzeit bereitstellen kann.

13. Verfahren nach einem der vorhergehenden Ansprüche 11 bis 12, wobei die Augendeskriptoren des Arrays aus einer Liste ausgewählt sind, bestehend aus: einer Pupillenebene, einem 2D-Pupillenzentrum, einem Konfidenzwert für das 2D-Pupillenzentrum, einem Hauptradius der 2D-Pupillenellipse, einem Konfidenzwert für den Hauptradius der 2D-Pupillenellipse, einem Nebenradius der 2D-Pupillenellipse, einem Konfidenzwert für den Nebenradius der 2D-Pupillenellipse, einer Orientierung der 2D-Pupillenellipse, einem Konfidenzwert für die Orientierung der 2D-Pupillenellipse, einem Pupillenzentrumsstrahl, einem projizierten Pupillenzentrumsstrahl, einer 3D-Extrusion des projizierten Pupillenzentrumsstrahls, einer Tiefenebene, einer Augapfelzentrumsebene, einem 3D-Augapfelzentrum, einem Konfidenzwert für das 3D-Augapfelzentrum, einem 3D-Pupillenzentrum, einem Konfidenzwert für das 3D-Pupillenzentrum, einem Abstand zwischen dem Augapfelzentrum und einer Augenkamera, einem Augapfelradius, einer optischen Achse, einer visuellen Achse, einer Blicklinie, einer 3D-Blickrichtung, einer monokularen 3D-Blickrichtung, einem Konfidenzwert für die monokulare 3D-Blickrichtung, einem Vergenzwinkel, einem binokularen 3D-Blickpunkt, einem Konfidenzwert für den binokularen 3D-Blickpunkt, einem binokularen 2D-Blickpunkt, einem Konfidenzwert für den binokularen 2D-Blickpunkt, einem Pupillendurchmesser, einem Konfidenzwert für den Pupillendurchmesser, einem scheinbaren Pupillendurchmesser, einer Pupillenfläche, einem Irisdurchmesser, einem Konfidenzwert für den Irisdurchmesser, einem Hornhautlimbus, einem Hauptradius des Hornhautlimbus, einem Konfidenzwert für den Hauptradius des Hornhautlimbus, einem Nebenradius des Hornhautlimbus, einem Konfidenzwert für den Nebenradius des Hornhautlimbus, einer Orientierung des Hornhautlimbus, einem Konfidenzwert für die Orientierung des Hornhautlimbus, einem Abstand zwischen dem Zentrum des Hornhautlimbus und dem Pupillenzentrum, einer Krümmung der vorderen Hornhautoberfläche ausgedrückt in keratometrischen Dioptrien, einem Abstand zwischen dem Hornhautapex und dem Pupillenzentrum, einem Abstand zwischen dem Hornhautvertex und dem Pupillenzentrum, einem Brechungsindex der Hornhaut, einem Brechungsindex der Vorderkammer des Auges, einem Brechungsindex der Hinterkammer des Auges, einem Brechungsindex der Augenlinse, einem Abstand zwischen der Augenlinse und dem 3D-Augapfelzentrum, einer Sklera mit bestimmten Lichtstreueigenschaften, einer Klassifizierung des Augenzustands offen/geschlossen, einer Winkelgeschwindigkeit des Augapfels, einer Winkelbeschleunigung des Augapfels, Statistiken zu Augenbewegungen, einer Klassifizierung des binokularen Blicks in Blinzel/Fixations-/Folgebewegungs-/Sakkaden-/vestibulo-okulären-Reflex-/Mikrosakkaden-Ereignisse, Statistiken zur Bewertung der kognitiven Belastung des Benutzers, Statistiken zur Bewertung der Aufmerksamkeit des Benutzers, Statistiken zur Bewertung des Bewusstseinszustands des Benutzers sowie Augenmerkmalen und Augenstatistiken des Benutzers zur Benutzeridentifizierung und/oder Benutzerauthentifizierung.

14. Verfahren nach einem der vorhergehenden Ansprüche 11 bis 13, wobei mindestens ein Augendeskriptor durch mindestens einen Schritt eines neuronalen Netzes inferiert wird.

15. Verfahren nach Anspruch 14, wobei der mindestens eine Schritt des neuronalen Netzes eine Architektur eines neuronalen Netzes zur Lokalisierung von mindestens einem Augenmerkmal des Benutzers in den Eingabe-Augendarstellungen des Benutzers implementiert, wobei die Eingaben und/oder Ausgaben des Schritts des neuronalen Netzes und/oder die Gewichte des Schritts des neuronalen Netzes auf 8 Bit oder weniger quantisiert sind und/oder die Aktivierungen des Schritts des neuronalen Netzes auf 16 Bit oder weniger quantisiert sind.

## Revendications

1. Dispositif porté sur la tête (2), mettant en œuvre une unité de descripteurs oculaires (1) pour estimer le regard de l'utilisateur une fois que l'unité de descripteurs oculaires (1) est connectée au dispositif porté sur la tête (2) porté par un utilisateur lors de l'utilisation, ladite unité de descripteurs oculaires (1) comprenant une première partie de pont nasal en forme de U réalisée sur circuit imprimé (30), configurée pour être intégrée dans une partie de pont nasal portable en forme de U du dispositif porté sur la tête (2), compatible avec le nez d'un porteur, ladite première partie de pont nasal en forme de U sur circuit imprimé (30) comprenant au moins un capteur oculaire (12) orienté vers l'œil gauche de l'utilisateur et un capteur oculaire (12) orienté vers l'œil droit de l'utilisateur, ladite unité de descripteurs oculaires (1) comprenant une seconde partie sur circuit imprimé (31) comprenant une unité d'edge computing (10) reliée électroniquement aux capteurs oculaires (12) par un circuit électronique, ladite unité d'edge computing (10) étant munie d'un accélérateur matériel d'apprentissage automatique et configurée pour recevoir en entrée des données de représentation oculaire de l'utilisateur provenant des capteurs oculaires (12) et fournir en sortie un ensemble de descripteurs oculaires de l'utilisateur lors de l'utilisation, et dans lequel l'unité d'edge computing (10) est un microcontrôleur programmé avec un code bare metal personnalisé, ou avec un système d'exploitation temps réel RTOS pour microcontrôleurs, ou avec une combinaison d'un code bare metal personnalisé et d'un système d'exploitation temps réel RTOS pour microcontrôleurs.

2. Dispositif porté sur la tête (2) selon la revendication 1, dans lequel la partie de pont nasal portable en forme de U du dispositif porté sur la tête est conçue de manière que le capteur oculaire (12) soit intégré dans les branches de la partie de pont nasal (42), sans qu'aucune partie saillante de la monture renfermant le capteur oculaire (12) n'interfère avec le champ de vision de l'utilisateur lors de l'utilisation.

3. Dispositif porté sur la tête (2) selon l'une quelconque des revendications précédentes, dans lequel un angle β est compris dans la plage 0 degré ≤ β ≤ 40 degrés, de préférence dans la plage 0 degré ≤ β ≤ 20 degrés ± 2 degrés en tant que tolérance de fabrication et d'assemblage, ladite plage étant définie par les valeurs absolues de β par rapport à l'axe Z, ledit angle β étant projeté sur le plan YZ qui constitue le plan de symétrie du dispositif porté sur la tête (2), en supposant un système de référence (X, Y, Z) dont l'origine correspond au point du pont nasal (O), le plan XZ du système de référence passant par le point nasal (O), défini comme le point médian le plus élevé du bord inférieur du pont nasal par rapport à la section transversale dans le plan de symétrie du dispositif porté sur la tête (2), et par les points de branche (E100, ... , E125), définis comme le point du bord inférieur des branches (11) supposé être en contact avec le sommet de l'oreille lors de l'utilisation, lesdits points de branche (E100, ... , E125) étant situés à une distance de 100 mm, 105 mm, 110 mm, 115 mm, 120 mm ou 125 mm, ou étant l'un quelconque des points situés à une distance du point du pont nasal (O) comprise entre 100 mm et 125 mm, mesurée à partir du point du pont nasal (O) le long de l'axe Z.

4. Dispositif porté sur la tête (2) selon la revendication 3, dans lequel le capteur oculaire gauche (12) et le capteur oculaire droit (12) sont disposés dans la monture de partie nasale correspondante (42) à une position ayant une coordonnée Y par rapport à l'axe Y du système de référence dans la plage : Ŷ - 5 mm ≤ Y ≤ Ŷ + 5 mm, où Y < 0 et où - Ŷ = δ + λ * tan β, avec δ = 3.4 mm et λ = 19.5 mm.

5. Dispositif porté sur la tête (2) selon l'une quelconque des revendications précédentes, dans lequel un angle α est compris dans la plage 20 degrés ≤ α ≤ 50 degrés ± 2 degrés en tant que tolérance de fabrication et d'assemblage, ladite plage étant définie par des valeurs absolues par rapport à l'axe Z dans le plan ZX, ledit angle α étant situé dans le plan ZX passant par le point du pont nasal (O), défini comme le point médian le plus élevé du bord inférieur du pont nasal par rapport à la section transversale dans le plan de symétrie du dispositif porté sur la tête (2), et par les points de branche (E100, ... , E125), définis comme le point du bord inférieur de la branche (11) supposé être en contact avec le sommet de l'oreille lors de l'utilisation, lesdits points de branche (E100, ... , E125) étant situés à une distance de 100 mm, 105 mm, 110 mm, 115 mm, 120 mm ou 125 mm, ou étant l'un quelconque des points situés à une distance du point du pont nasal (O) comprise entre 100 mm et 125 mm, mesurée à partir du point du pont nasal (O) le long de l'axe Z.

6. Dispositif porté sur la tête (2) selon l'une quelconque des revendications précédentes, dans lequel la monture du dispositif porté sur la tête (2) comprend une partie supérieure de monture (41), une partie de pont nasal portable en forme de U (42) reliée au centre de la partie supérieure de monture (41), ainsi qu'une partie latérale gauche et une partie latérale droite de monture (40), la combinaison desdites parties de monture étant configurée pour maintenir les verres en place et rendre les verres remplaçables lorsqu'ils sont disposés sur le dispositif porté sur la tête (2).

7. Dispositif porté sur la tête (2) selon l'une quelconque des revendications précédentes, dans lequel l'unité de descripteurs oculaires (1) comprend une LED infrarouge disposée dans la première partie de pont nasal en forme de U sur circuit imprimé (30).

8. Dispositif porté sur la tête (2) selon l'une quelconque des revendications précédentes, dans lequel l'unité de descripteurs oculaires (1) comprend une caméra frontale destinée à capturer le champ de vision de l'utilisateur lors de l'utilisation.

9. Dispositif porté sur la tête (2) selon l'une quelconque des revendications précédentes, dans lequel la monture du pont nasal (42) est formée d'au moins deux parties principales, à savoir un support et un couvercle, ledit support et ledit couvercle constituant chacun une structure monobloc fabriquée par un procédé de fabrication additive.

10. Dispositif porté sur la tête (2) selon la revendication 9, dans lequel le support est constitué d'un matériau additif massif duquel n'est soustrait que l'espace destiné à l'unité de descripteurs oculaires (1) et à ses connexions électroniques avec le dispositif porté sur la tête (2).

11. Procédé de détermination d'un ensemble de données de regard, le procédé étant mis en œuvre à l'aide du dispositif porté sur la tête (2) selon l'une quelconque des revendications précédentes, le dispositif porté sur la tête (2) étant configuré pour :
- recevoir en entrée au moins deux données de représentation oculaire de l'utilisateur à deux instants différents provenant des capteurs oculaires (12),
- estimer la géométrie oculaire de l'utilisateur sur la base de l'entrée, et
- fournir en sortie l'ensemble de descripteurs oculaires de l'utilisateur.

12. Procédé selon la revendication 11, dans lequel le temps d'inférence de l'ensemble de caractéristiques oculaires de l'utilisateur est inférieur au temps d'acquisition des données de représentation oculaire de l'utilisateur à partir du capteur oculaire (12), permettant au procédé de fournir des caractéristiques oculaires de l'utilisateur en temps réel.

13. Procédé selon l'une quelconque des revendications 11 à 12, dans lequel les descripteurs oculaires de l'ensemble sont sélectionnés dans une liste comprenant : un plan pupillaire, un centre pupillaire 2D, une valeur de confiance du centre pupillaire 2D, un grand rayon de l'ellipse pupillaire 2D, une valeur de confiance du grand rayon de l'ellipse pupillaire 2D, un petit rayon de l'ellipse pupillaire 2D, une valeur de confiance du petit rayon de l'ellipse pupillaire 2D, une orientation de l'ellipse pupillaire 2D, une valeur de confiance de l'orientation de l'ellipse pupillaire 2D, un rayon du centre pupillaire, un rayon projeté du centre pupillaire, une extrusion 3D du rayon projeté du centre pupillaire, un plan de profondeur, un plan du centre du globe oculaire, un centre du globe oculaire 3D, une valeur de confiance du centre du globe oculaire 3D, un centre pupillaire 3D, une valeur de confiance du centre pupillaire 3D, une distance entre le centre du globe oculaire et une caméra oculaire, un rayon du globe oculaire, un axe optique, un axe visuel, une ligne de visée, une direction de regard 3D, une direction de regard 3D monoculaire, une valeur de confiance de la direction de regard 3D monoculaire, un angle de vergence, un point de regard 3D binoculaire, une valeur de confiance du point de regard 3D binoculaire, un point de regard 2D binoculaire, une valeur de confiance du point de regard 2D binoculaire, un diamètre pupillaire, une valeur de confiance du diamètre pupillaire, un diamètre pupillaire apparent, une aire pupillaire, un diamètre de l'iris, une valeur de confiance du diamètre de l'iris, un limbe cornéen, un grand rayon du limbe cornéen, une valeur de confiance du grand rayon du limbe cornéen, un petit rayon du limbe cornéen, une valeur de confiance du petit rayon du limbe cornéen, une orientation du limbe cornéen, une valeur de confiance de l'orientation du limbe cornéen, une distance entre le centre du limbe cornéen et le centre pupillaire, une courbure de la surface antérieure de la cornée exprimée en dioptries kératométriques, une distance entre l'apex cornéen et le centre pupillaire, une distance entre le vertex cornéen et le centre pupillaire, un indice de réfraction de la cornée, un indice de réfraction de la chambre antérieure de l'œil, un indice de réfraction de la chambre postérieure de l'œil, un indice de réfraction du cristallin, une distance entre le cristallin et le centre du globe oculaire 3D, une sclère présentant certaines propriétés de diffusion lumineuse, une classification de l'état œil ouvert/œil fermé, une vitesse angulaire du globe oculaire, une accélération angulaire du globe oculaire, des statistiques de mouvements oculaires, une classification du regard binoculaire en événements de type clignement/fixation/poursuite/saccade/réflexe vestibulo-oculaire/microsaccade, des statistiques pour l'évaluation de la charge cognitive de l'utilisateur, des statistiques pour l'évaluation de l'attention de l'utilisateur, des statistiques pour l'évaluation de la conscience de l'utilisateur, ainsi que des caractéristiques oculaires et statistiques oculaires de l'utilisateur pour l'identification et/ou l'authentification de l'utilisateur.

14. Procédé selon l'une quelconque des revendications 11 à 13, dans lequel au moins un descripteur oculaire est inféré par au moins une étape de réseau neuronal.

15. Procédé selon la revendication 14, dans lequel ladite au moins une étape de réseau neuronal met en œuvre une architecture de réseau neuronal pour localiser au moins une caractéristique oculaire de l'utilisateur dans l'entrée de représentation oculaire de l'utilisateur, dans lequel l'entrée et/ou les sorties de l'étape de réseau neuronal et/ou les poids de l'étape de réseau neuronal sont quantifiés à 8 bits ou moins et/ou les activations de l'étape de réseau neuronal sont quantifiées à 16 bits ou moins.
